# EUROPÄISCHE PATENTSCHRIFT

(11) **EP 3 125 805 B1**
(45) Veröffentlichungstag und Bekanntmachung des Hinweises auf die Patenterteilung: **06.06.2018**
(21) Anmeldenummer: 15712875.2
(22) Anmeldetag: 25.03.2015
(51) Int. Cl.: A61B 90/00, A61B 34/00

(54) **MEDIZINISCHE BEFESTIGUNGSEINRICHTUNG SOWIE REFERENZIERUNGSVORRICHTUNG UND MEDIZINISCHES INSTRUMENTARIUM**
MEDICAL FIXATION DEVICE, REFERENCING DEVICE AND MEDICAL INSTRUMENT
DISPOSITIF MÉDICAL DE FIXATION, DISPOSITIF DE RÉFÉRENCE ET INSTRUMENT MÉDICAL

(30) Priorität: 03.04.2014 DE 102014104800
(43) Veröffentlichungstag der Anmeldung: 08.02.2017
(73) Patentinhaber: Aesculap AG, 78532 Tuttlingen (DE)
(72) Erfinder: GASSNER, Stefan, 78194 Immendingen-Hattingen (DE); ALTMANN, Steffi, 78166 Donaueschingen (DE); MÜLLER, Markus, 72461 Albstadt (DE)
(74) Vertreter: Hoeger, Stellrecht & Partner Patentanwälte mbB
(86) Internationale Anmeldenummer: PCT/EP2015/056383
(87) Internationale Veröffentlichungsnummer: WO 2015/150185

(56) Entgegenhaltungen:
- EP-A1- 2 198 801
- WO-A1-01/21084
- WO-A1-2009/059434
- CN-A- 102 258 399
- DE-U1- 20 315 470
- GB-A- 2 391 814
- US-A- 4 492 227
- US-A- 6 050 967
- US-A1- 2009 018 445

## Beschreibung

Die Erfindung betrifft eine medizinische Befestigungseinrichtung zum nichtinvasiven Befestigen einer zwei oder mehr Markierelemente aufweisenden medizinischen Markiereinrichtung an einem Körperteil eines Patienten, insbesondere an einem Oberschenkel, wobei die Befestigungseinrichtung einen in zwei im Winkel zueinander ausgerichteten Erstreckungsrichtungen flächig erstreckten, in seiner Form an die Kontur des Körperteils anpassbaren Trägerkörper umfasst, an dem die Markiereinrichtung festgelegt oder festlegbar ist, wobei der Trägerkörper in der Fläche seiner Erstreckung elastisch dehnbar ist und einen ersten Dehnungsbereich und zumindest einen zweiten Dehnungsbereich umfasst, dessen Dehnbarkeit in zumindest einer Richtung in der Fläche der Erstreckung des Trägerkörpers geringer ist als diejenige des ersten Dehnungsbereiches.

Außerdem betrifft die Erfindung eine medizinische Referenzierungsvorrichtung mit einer Befestigungseinrichtung und einer Markiereinrichtung.

Ferner betrifft die Erfindung ein medizinisches Instrumentarium.

Es sind medizinische Referenzierungsvorrichtungen bekannt, die beispielsweise in der Knieprothetik zum Einsatz kommen, um einem Operateur die Implantation eines künstlichen Kniegelenkes zu erleichtern. Die Referenzierungsvorrichtung umfasst die Markiereinrichtung mit starr relativ zueinander angeordneten Markierelementen (sogenannter "Rigid Body"), deren Bewegung im Raum von einem Navigationssystem verfolgt wird. Um eine möglichst hohe Genauigkeit zu erzielen, wird die Markiereinrichtung über eine Befestigungseinrichtung, üblicherweise eine Knochenschraube, am Femurknochen oder am Tibiaknochen verschraubt und dadurch fest an diesen fixiert. Dies führt jedoch zu einem Eingriff nicht unerheblicher Invasivität, gegenüber dem bei einigen Operateuren und Patienten Vorbehalte bestehen.

Zur Verringerung der Invasivität ist es bekannt, Rigid Bodies an einer um den Femur (vorliegend Oberschenkel) oder die Tibia (vorliegend Unterschenkel) gelegten Manschette zu fixieren oder an einer auf den Femur oder die Tibia aufgelegten Platte anzubringen, die mit Bändern am jeweiligen Körperteil fixiert wird. Die Manschette umfasst oder bildet eine Befestigungseinrichtung mit einem flächig erstreckten Trägerkörper, der von seiner ursprünglich planaren Form verformt und an die Kontur des Körperteils (beispielsweise des Femurs oder der Tibia) angepasst werden kann, um einen formschlüssigen Sitz am Körperteil zu ermöglichen.

Bekannt sind auch Trägerkörper, die auf das Körperteil aufgeklebt werden können und an denen die Markiereinrichtung festgelegt ist.

Bei derartigen nichtinvasiven Befestigungseinrichtungen ergibt sich in der Praxis das Problem, dass Bewegungen der Haut und von Muskeln sowie Weichteilverschiebungen, insbesondere bei einer zu erfassenden Bewegung des Körperteils im Raum, über den Trägerkörper auf die Markiereinrichtung übertragen werden. Die Genauigkeit der Positionsdaten der Markiereinrichtung - und damit des Körperteils -, die der navigationsunterstützten Operation zugrunde gelegt werden soll, wird dadurch beeinträchtigt.

Wünschenswert wäre es außerdem, über eine nichtinvasiv am Körperteil anbringbare Befestigungseinrichtung prä- und postoperativ Patientendaten mit einem medizinischen Navigationssystem zu erfassen. Dadurch können Operationsdauern reduziert werden, weil hierfür erforderliche Daten bereits präoperativ zur Verfügung stehen und nicht erst intraoperativ ermittelt werden müssen. Postoperativ kann eine einfache Kontrolle des Erfolges der Operation durchgeführt werden. In beiden Fällen können ansonsten eingesetzte ionisierende Bildgebungsverfahren wie Röntgen oder CT ersetzt werden.

In der US 4,492,227 ist eine elastische Kniebandage mit einem schlauchförmigen, mindestens teilweise aus elastischem Textilmaterial bestehenden Grundkörper beschrieben, der einen am Kniegelenk anzulegenden Mittelbereich umfasst sowie zwei beidseits dieses Mittelbereichs angrenzende Randabschnitte, deren Dehnungseigenschaften sich von der des Mittelbereichs unterscheiden.

Eine Kompressionsbandage mit visueller Anzeige der Kompressionskraft ist in der US 6,050,967 beschrieben. Wird die Bandage unter Spannung gesetzt, verformt sich ein regelmäßiges Muster der Bandage, woraus die Kompressionskraft abgeleitet werden kann.

Die EP 2 198 801 A1 beschreibt eine Marker-Anbringvorrichtung zum Anbringen einer Markervorrichtung an einem Gegenstand, zum Beispiel einem medizinischen Instrument, einer Körperstruktur oder einem Implantat. Es ist eine Befestigungsmöglichkeit für eine Markervorrichtung und ein Anbringteil zum Anbringen der Marker-Anbringvorrichtung vorgesehen, wobei das Anbringteil verformbar ist, um mit dem Gegenstand eine mechanische Verbindung einzugehen.

Eine Vorrichtung zum nichtinvasiven Tracken einer medizinischen Markiereinrichtung ist in der CN 102258399 A beschrieben.

Die US 2009/0018445 A1 beschreibt eine Vorrichtung zum Tracken medizinischer Instrumente, u. a. einer Ultraschallsonde. Weitere Anwendungen medizintechnischen Markertrackings sind in der DE 203 15 470 U1, der WO 01/21084 A1 und der GB 2 391 814 A offenbart.

Aufgabe der vorliegenden Erfindung ist es, eine gattungsgemäße Befestigungseinrichtung, eine Referenzierungsvorrichtung und ein Instrumentarium bereitzustellen, unter deren bzw. dessen Einsatz die nichtinvasiv gewonnen Positionsdaten eine höhere Genauigkeit aufweisen.

Diese Aufgabe wird durch eine erfindungsgemäße Befestigungseinrichtung mit den Merkmalen von Anspruch 1 gelöst.

Der Trägerkörper der erfindungsgemäßen Befestigungseinrichtung ist elastisch dehnbar, wodurch er unter Spannung gesetzt am Körperteil (beispielsweise dem Femur oder der Tibia) befestigt werden kann. Wie sich in der Praxis zeigt, können dadurch die Haut, Weichteile und Muskeln unter der Dehnung des Trägerkörpers komprimiert werden. Dies gibt die Möglichkeit, eine Relativbewegung der Körperteile untereinander zu verringern. Die vom Körperteil auf den Trägerkörper und damit auf die Markiereinrichtung induzierte Bewegung kann dadurch ebenfalls verringert werden. Darüber hinaus umfasst der Trägerkörper einen ersten und mindestens einen zweiten Dehnungsbereich, die sich hinsichtlich ihrer Dehnbarkeit in zumindest einer Richtung in der Fläche der Erstreckung des Trägerkörpers unterscheiden. Durch Vorsehen unterschiedlich dehnbarer Dehnungsbereiche ist die Möglichkeit gegeben, die Haut, Weichteile und Muskeln mit unterschiedlicher Kompression zu beaufschlagen. Wie sich in der Praxis zeigt, kann die Induzierung von Bewegungen dieser Körperteile untereinander auf den Trägerkörper dadurch erheblich verringert werden. Außerdem können Bewegungen von Haut, Muskeln und Weichteilen untereinander im Wesentlichen innerhalb des Trägerkörpers absorbiert werden. Die Genauigkeit der gewonnen Daten kann dadurch gesteigert werden, indem die Markiereinrichtung an einem Abschnitt des Trägerkörpers festgelegt ist, der vom Operateur als einer geringen Eigenbewegung unterworfen erkannt wird. Dadurch kann eine stabile Referenz der Markiereinrichtung zum Knochen, beispielsweise dem Femurknochen oder dem Tibiaknochen, erzielt werden. Hierzu kann sich der Operateur vorzugsweise technischer Hilfsmittel bedienen, worauf nachfolgend noch eingegangen wird.

Die Befestigungseinrichtung umfasst mindestens ein Halteteil, das am Trägerkörper festgelegt ist, und die Markiereinrichtung oder ein Markierelement derselben ist an einer dem Trägerkörper abgewandten Seite des Haltteils bevorzugt lösbar kraft- und/oder formschlüssig festgelegt oder festlegbar. Das Halteteil kann am ersten und/oder am mindestens einen zweiten Dehnungsbereich festgelegt sein.

Vorzugsweise ist die Dehnbarkeit des mindestens einen zweiten Dehnungsbereiches in zwei im Winkel und insbesondere quer zueinander ausgerichteten Richtungen in der Fläche der Erstreckung des Trägerkörpers geringer als diejenige des ersten Dehnungsbereiches. Die Unterschiede der Dehnbarkeiten der Dehnungsbereiche können dadurch gesteigert werden. Wie sich in der Praxis zeigt, kann dadurch die Genauigkeit der gewonnenen Positionsdaten erhöht werden.

Selbstverständlich kann vorgesehen sein, dass die Dehnbarkeit des mindestens einen zweiten Dehnungsbereiches in einer Richtung in der Fläche der Erstreckung des Trägerkörpers geringer ist als die Dehnbarkeit des ersten Dehnungsbereiches, welche Richtung in einem Winkel zu den beiden Richtungen bei der zuletzt erwähnten vorteilhaften Ausführungsform der Befestigungseinrichtung ausgerichtet ist, insbesondere welche Richtung eine Überlagerung bei den zwei Richtungen der zuletzt genannten Ausführungsform ist.

Die mindestens eine Richtung in der Fläche der Erstreckung des Trägerkörpers, in der der mindestens eine zweite Dehnungsbereich eine geringere Dehnbarkeit aufweist als der erste Dehnungsbereich, ist beispielsweise eine der beiden Erstreckungsrichtungen des Trägerkörpers. Bei Anlage der Befestigungseinrichtung am Oberschenkel oder an der Tibia, die zu einer Verformung des Trägerkörpers führt, verläuft die erste Erstreckungsrichtung beispielsweise in Umfangsrichtung des Oberschenkels bzw. der Tibia. Eine quer zur ersten Erstreckungsrichtung ausgerichtete zweite Erstreckungsrichtung des Trägerkörpers kann bei Anlage der Befestigungseinrichtung am Oberschenkel oder an der Tibia beispielsweise in dessen/deren Längsrichtung verlaufen.

Bei einer vorteilhaften Ausführungsform der erfindungsgemäßen Befestigungseinrichtung ist genau ein zweiter Dehnungsbereich vorgesehen. Dementsprechend kann der Trägerkörper einen ersten Dehnungsbereich und einen zweiten Dehnungsbereich umfassen.

Als günstig erweist es sich, wenn der erste Dehnungsbereich und/oder der mindestens eine zweite Dehnungsbereich in sich zusammenhängend ist bzw. sind.

Bei einer andersartigen vorteilhaften Ausführungsform der Befestigungseinrichtung kann vorgesehen sein, dass der erste Dehnungsbereich und/oder der mindestens eine zweite Dehnungsbereich räumlich voneinander getrennte Abschnitte aufweisen können.

Bei einer günstigen Umsetzung der Befestigungseinrichtung in der Praxis ist es von Vorteil, wenn der mindestens eine zweite Dehnungsbereich den ersten Dehnungsbereich umgibt. Beispielsweise bildet der mindestens eine zweite Dehnungsbereich eine Einfassung für den ersten Dehnungsbereich in der Fläche des Trägerkörpers. Der mindestens eine zweite Dehnungsbereich kann insbesondere einen Rand des Trägerkörpers bilden, der den ersten Dehnungsbereich einfasst.

Der Trägerkörper kann in Draufsicht eine rechteckige oder im Wesentlichen rechteckige Kontur aufweisen. Beispielsweise ist dies dann der Fall, wenn der Trägerkörper ein Abschnitt oder Bestandteil einer von der Befestigungseinrichtung ausgestalteten Bandage ist, worauf nachfolgend noch eingegangen wird.

Bei einer vorteilhaften Umsetzung der Befestigungseinrichtung erweist es sich als günstig, wenn der erste Dehnungsbereich in Draufsicht auf die Fläche des

Trägerkörpers eine rechteckige oder im Wesentlichen rechteckige Kontur aufweist. Dies ist insbesondere dann von Vorteil, wenn der mindestens eine zweite Dehnungsbereich, wie vorstehend erwähnt, den ersten Dehnungsbereich einfasst und einen Rand des Trägerkörpers bildet.

Bei einer andersartigen Umsetzung der Befestigungseinrichtung ist es von Vorteil, wenn der erste Dehnungsbereich in Draufsicht auf die Fläche des Trägerkörpers eine taillierte Kontur aufweist, mit zwei Verbreiterungsabschnitten und einem dazwischen angeordneten Verschmälerungsabschnitt. Eine derartige Ausgestaltung der Befestigungseinrichtung kommt zum Beispiel bei einer Oberschenkel-Befestigungseinrichtung zum Einsatz, insbesondere in Form einer Bandage. Die Orientierung des ersten Dehnungsbereiches mit Verbreiterungsabschnitt-Verschmälerungsabschnitt-Verbreiterungsabschnitt ist dabei vorzugsweise in Längsrichtung des Femurs ausgerichtet.

Als besonders günstig erweist es sich, wenn der Trägerkörper ein Textilverbund in Form eines Gestricks, eines Gewirks oder eines Gewebes ist oder wenn der Trägerkörper einen solchen Textilverbund umfasst. Dies erlaubt es, die Dehnbarkeit des ersten und des mindestens zweiten Dehnungsbereiches herstellungstechnisch einfach bereitzustellen und voneinander unterschiedlich auszugestalten. Fadenförmiges Ausgangsmaterial für den Textilverbund kann Natur- und/oder Kunstfasern und/oder elastische Fäden umfassen oder daraus bestehen.

Von Vorteil ist es, wenn der erste Dehnungsbereich und der mindestens eine zweite Dehnungsbereich miteinander durch Stricken, Wirken oder Weben verbunden sind und dementsprechend miteinander verstrickt, verwirkt oder verwebt sind. Dies ermöglicht es, die Dehnungsbereiche und den Trägerkörper in einem einzigen, einheitlichen Herstellungsverfahren zu stricken, wirken oder weben. Es ist insbesondere nicht erforderlich, die Dehnungsbereiche getrennt voneinander herzustellen und auf andersartige Weise miteinander zu verbinden, beispielsweise durch Nähen.

Bevorzugt ist die unterschiedliche Dehnbarkeit des ersten Dehnungsbereiches und des mindestens einen zweiten Dehnungsbereiches durch die Art des Gestricks, des Gewirks oder des Gewebes bereitgestellt, insbesondere durch die Verknüpfungsart und/oder die Anzahl und/oder Beschaffenheit der Fäden des Textilverbundes. So können für den ersten und den mindestens einen zweiten Dehnungsbereich unterschiedliche Bindungen zum Einsatz kommen, bei denen sich die Verknüpfungen der Fäden voneinander unterscheiden, wodurch die Dehnbarkeit beeinflusst werden kann. Auch die Fadendichte, das eingesetzte Fadenmaterial, speziell die Fadendehnbarkeit und/oder die Anzahl eingesetzter Fäden kann - beispielhaft und nichtabschließend - in den Dehnungsbereichen unterschiedlich sein, um deren Dehnbarkeit zu beeinflussen.

Bei einer Umsetzung der Befestigungseinrichtung in der Praxis erweist es sich als günstig, wenn der erste Dehnungsbereich ein Gestrick mit Köperbindung (speziell mit elastischen Fäden) oder einer anderen Bindung mit hoher Zweizugelastizität (z. B. Fangbindung) ist oder umfasst und/oder wenn der mindestens eine zweite Dehnungsbereich ein Gestrick mit Halbschlauchbindung oder einer anderen Bindung mit geringer Zweizugelastizität (z. B. Rechts-Rechts-Bindung ohne elastischen Faden, Jacquardbindung, Milano-Ripp-Bindung) ist oder umfasst. Eine hohe Dehnbarkeit des ersten Dehnungsbereiches kann beispielsweise durch Einsatz elastisch dehnbarer Strickfäden am ersten Dehnungsbereich erzielt werden.

Von Vorteil ist es, wenn die Befestigungseinrichtung als Bandage ausgestaltet ist oder eine solche umfasst, die von einem geöffneten Lösezustand in einen in sich geschlossenen Anlegezustand überführbar ist und die ein oder mehrere Fixierelemente zum Fixieren der Bandage im Anlegezustand aufweist. Der Trägerkörper kann einen Abschnitt der Bandage bilden, an dem zum Beispiel Fixierelemente befestigt sind, mit denen die Bandage im geschlossenen, ringförmigen Anlegezustand fixierbar ist. Die Fixierelemente umfassen beispielsweise einen Gurt, eine Schlaufe oder Öse, durch die der Gurt hindurchführbar ist, um die Bandage zu straffen oder eine Schnalle zu demselben Zweck. Weitere Beispiele für Fixierelemente sind Druckknöpfe, ein Klettverschluss oder eine Klebeschicht, beispielsweise um den Gurt in sich zu fixieren. Eine vorteilhafte Umsetzung umfasst einen Gurt und eine Schlaufe, damit die Bandage unter Dehnung des ersten und des mindestens einen zweiten Dehnungsbereiches straff am Körperteil, insbesondere dem Femur, angelegt werden kann.

Vorteilhafterweise ist die Länge der Bandage im Anlegezustand für einen formschlüssigen Sitz am Körperteil anpassbar. Zu diesem Zweck können beispielsweise Fixierelemente in Form eines Gurtes und einer Schlaufe zum Hindurchführen des Gurtes zusammenwirken. Der Gurt kann in sich beispielsweise mittels eines Klettverschlusses fixiert werden.

Bei einer andersartigen vorteilhaften Umsetzung ist es günstig, wenn die Befestigungseinrichtung als in sich geschlossene Schlauchbandage ausgestaltet ist oder eine solche umfasst. Die Schlauchbandage, die ganz oder abschnittsweise vom Trägerkörper gebildet ist, kann unter Dehnung aufgeweitet und über das Körperteil gestreift werden. Dies gibt die Möglichkeit, die Schlauchbandage formschlüssig am Körperteil zu fixieren und dieses zu komprimieren.

Die Bandage ist vorzugsweise eine Oberschenkelbandage. Die Oberschenkelbandage kann an einem Rand eine Ausnehmung aufweisen, in die die Kniescheibe oder darüber angeordnetes Weichteilgewebe eingreifen kann. Dies ermöglicht es, die Oberschenkelbandage in definierter Weise relativ zum Körperteil, hier dem Femur, auszurichten. Am Rand der Ausnehmung kann ein Abschnitt geringer Verformbarkeit der Oberschenkelbandage angeordnet sein. Dieser Abschnitt kann bei angelegter Bandage oberhalb der Sehne des Musculus quadriceps positioniert sein. An dieser Stelle wird vorzugsweise die Markiereinrichtung oder ein Markierelement derselben am Trägerkörper festgelegt, da die Bewegung innerhalb des Trägerkörpers oberhalb der Sehne des Musculus quadriceps gering ist und eine Übertragung einer Bewegung von Körperteilen auf die Markiereinrichtung gering gehalten werden kann.

Die Befestigungseinrichtung kann auch eine folienförmige Ausgestaltung aufweisen. Beispielsweise kann der Trägerkörper auf den Patientenkörper aufklebbar sein.

Günstig ist es, wenn der Trägerkörper auf seiner dem Körperteil zugewandten Seite Reibwertsteigerungselemente umfasst. Die Reibwertsteigerungselemente können ungleichmäßig am Trägerkörper angeordnet sein. Dies erlaubt es, über die Reibwertsteigerungselemente Haut, Muskeln oder Weichteile zusätzlich zu straffen. Dies erweist sich insbesondere bei einer der vorstehend genannten Bandagen als vorteilhaft. Die Befestigungseinrichtung kann im Bereich der Reibwertsteigerungselemente zusätzlich am Körperteil stabilisiert werden, ohne selbst bei straff angelegter Befestigungseinrichtung eine Blutsperre am Körperteil zu erzeugen. Vorzugsweise ist es möglich, über die Reibwertsteigerungselemente Relativbewegungen der Körperteile zueinander gezielter in den Trägerkörper einzuleiten und darin zu absorbieren, um eine Übertragung von Bewegungen auf die Markiereinrichtung zu verringern.

Reibwertsteigerungselemente können am ersten Dehnungsbereich und/oder am zweiten Dehnungsbereich angeordnet sein.

Die Reibwertsteigerungselemente sind vorzugsweise aus einem Silikonmaterial gefertigt oder können ein solches umfassen.

Die Reibwertsteigerungselemente können unterschiedliche Formen und/oder unterschiedliche Größen aufweisen. Beispielsweise sind die Reibwertsteigerungselemente punktförmig, zum Beispiel ungefähr stecknadelkopfgroß.

Als Reibwertsteigerungselement kann mindestens ein Faden vorgesehen sein, der in den vorstehend genannten Textilverbund eingearbeitet ist, beispielsweise in ein Gestrick eingestrickt ist. Der Faden besteht beispielsweise aus einem Silikonmaterial.

Vorzugsweise sind entlang zumindest eines Randes oder parallel zu einem Rand des Trägerkörpers Reibwertsteigerungselemente angeordnet. Beispielsweise können bei einer Bandage in Umfangrichtung der Bandage, bezogen auf deren Anlegezustand, Reibwertsteigerungselemente am Rand des Trägerkörpers angeordnet sein.

Günstig ist es, wenn Reibwertsteigerungselemente an zwei einander gegenüberliegenden parallel zueinander verlaufenden Rändern des Trägerkörpers vorgesehen sind. Beispielsweise sind an einander gegenüberliegenden Rändern der Bandage längs deren Umfangrichtung im Anlegezustand Reibwertsteigerungselemente vorhanden.

Es kann alternativ oder ergänzend vorgesehen sein, dass an einem Rand zwei mit Reibwertsteigerungselementen versehende Abschnitte des Trägerkörpers vorgesehen sind, zwischen denen ein ohne Reibwertsteigerungselemente vorgesehener Abschnitt des Trägerkörpers angeordnet ist. Insbesondere kann ein Abschnitt des Trägerkörpers ohne Reibwertsteigerungselemente am Rand der vorstehend erwähnten Ausnehmung einer Bandage vorhanden sein.

Bei der zuletzt erwähnten vorteilhaften Ausführungsform kann die Reibung zwischen dem Körperteil, beispielsweise dem Oberschenkel, und dem Trägerkörper an dem Abschnitt ohne Reibwertsteigerungselemente verringert sein. Dies erweist sich beispielsweise bei einer Extension und einer Flexion des Beines als vorteilhaft. Im Bereich des Abschnittes wird der Trägerkörper dadurch in geringerem Maße von der Haut erfasst als ein mit Reibwertsteigerungselementen versehener Abschnitt. Der Abschnitt ohne Reibwertsteigerungselemente kann sich dadurch als so räumlich stabil am Oberschenkel erweisen, dass eine am Trägerkörper an dessen der Haut abgewandten Seite angeordnete Markiereinrichtung im Bereich dieses Abschnittes eine valide Referenz zum Femurknochen darstellen kann, weil sie auch bei Extension und Flexion des Beines die Position relativ zum Femurknochen beibehalten kann.

Das Halteteil ist wie vorstehend beschrieben im Fall einer Bandage bevorzugt an einem Rand oder nahe eines Randes einer Ausnehmung am Trägerkörper angeordnet. Günstigerweise ist das Halteteil oberhalb einer Sehne des Musculus quadriceps angeordnet, wenn die Bandage bestimmungsgemäß am Femur angelegt ist.

Bevorzugt greift das Halteteil mit einer dem Trägerkörper zugewandten Seite in den Trägerkörper ein oder ist in diesen eingebettet. Bei Vorhandensein eines Textilverbundes kann das Halteteil zum Beispiel in den Trägerkörper eingebettet und dadurch an diesem verankert sein.

Als vorteilhaft erweist es sich, wenn das Halteteil nicht-starr ist. Eine direkte Übertragung von Bewegungen des Trägerkörpers an die Markiereinrichtung oder das Markierelement kann dadurch vermieden werden.

Als günstig erweist es sich daher, wenn das mindestens eine Halteteil verformbar ausgestaltet ist, wobei vorzugsweise die Verformbarkeit an einer dem Trägerkörper zugewandten Seite des Halteteils größer ist als an einer dem Trägerkörper abgewandten Seite des Halteteils. Die Verformbarkeit des Halteteils kann dementsprechend vom Trägerkörper zur Markiereinrichtung oder dem Markierelement abnehmen und die Härte des Halteteils zunehmen. Bewegungen innerhalb des Trägerkörpers können dadurch vom Halteteil aufgenommen werden. Sie werden aber aufgrund dessen abnehmender Verformbarkeit nicht oder nur in geringem Umfang an die Markiereinrichtung oder das Markierelement übertragen, so dass deren bzw. dessen Bewegung vermindert und die Genauigkeit der Positionsdaten erhöht werden kann.

Es kann vorgesehen sein, dass das Halteteil einen Schichtaufbau aufweist mit zwei oder mehr übereinanderliegenden Schichten, wobei die Schichten eine unterschiedliche Verformbarkeit aufweisen. Eine der Schichten kann wie vorstehend erwähnt in den Trägerkörper eingebettet sein, zum Beispiel in einem Textilverbund. Die Schichten sind zum Beispiel aus einem Silikonmaterial gefertigt oder umfassen ein Silikonmaterial.

Alternativ oder ergänzend kann vorgesehen sein, dass das Halteteil eine poröse, Hohlräume umfassende Struktur aufweist, wobei eine mittlere Größe von Hohlräumen im Halteteil an dessen dem Trägerkörper abgewandten Seite geringer ist als an dessen dem Trägerkörper zugewandten Seite. Dadurch ist ebenfalls die Möglichkeit gegeben, das Halteteil nahe am Trägerkörper verformbarer auszugestalten als im Abstand zum Trägerkörper, wo die Markiereinrichtung oder das Markierelement angeordnet ist.

Es ist denkbar, dass die Markiereinrichtung oder das Markierelement unmittelbar am Halteteil angebracht ist.

Als vorteilhaft erweist es sich, wenn an der dem Trägerkörper abgewandten Seite des Halteteils ein Halteelement festgelegt oder in dieses eingebettet ist, an dem die Markiereinrichtung oder das Markierelement festgelegt oder festlegbar ist. Beispielsweise ist das Halteelement in eine der vorstehend genannten Schichten des Halteteils eingebettet. Das Halteelement kann mit einem korrespondierenden Befestigungselement an der Markiereinrichtung oder am Markierelement zusammenwirken, zum Beispiel durch Verschraubung, Verklemmung oder Verrastung. Auch eine Verbindung über Magnete, eine Klettverbindung oder durch Verkleben ist möglich. Insgesamt kann eine formschlüssige und/oder kraftschlüssige Befestigung der Markiereinrichtung oder des Markierelementes vorhanden sein.

Wie eingangs erwähnt, betrifft die Erfindung auch eine medizinische Referenzierungsvorrichtung. Eine gattungsgemäße medizinische Referenzierungsvorrichtung umfasst eine Markiereinrichtung mit mindestens zwei oder mehr chirurgischen Markierelementen, die zum Reflektieren und/oder Emittieren von Strahlung ausgebildet sind und eine Markierelementanordnung ausbilden zur Definition einer Referenz am Körper eines Patienten, sowie eine Befestigungseinrichtung, mit der die unter Einsatz der Befestigungseinrichtung erzielbaren Vorteile erzielbar sind.

Die eingangs genannte Aufgabe wird bei einer derartigen Referenzierungsvorrichtung erfindungsgemäß dadurch gelöst, dass die Befestigungseinrichtung eine Befestigungseinrichtung der vorstehend genannten Art ist und dass die Markiereinrichtung an der Befestigungseinrichtung, bevorzugt lösbar, festlegbar oder festgelegt ist.

Die Markierelementanordnung kann starr ausgebildet sind, wobei die Markierelemente fest miteinander verbunden sind und gemeinsam an der Befestigungseinrichtung festlegbar sind.

Alternativ kann die Markierelementanordnung nicht-starr ausgebildet sein, wobei zwei oder mehr Markierelemente positionsveränderlich zueinander sind. Beispielsweise sind die Markierelemente getrennt voneinander positionierbar, insbesondere individuell am Trägerkörper festlegbar.

Von Vorteil ist es, wenn die Markiereinrichtung ein möglichst geringes Gewicht und eine möglichst geringe Größe, insbesondere eine möglichst geringe Höhe relativ zum Körperteil, aufweist. Dadurch lässt sich die Genauigkeit der gewonnenen Positionsdaten erhöhen.

Beispielsweise kann vorgesehen sein, dass die Markiereinrichtung ein plattenförmiges Tragelement aufweist, an dem die Markierelemente festgelegt sind, wodurch die Markiereinrichtung eine geringe Bauhöhe aufweist. Der Abstand der Markiereinrichtung, an der Befestigungseinrichtung festgelegt, relativ zum zu referenzierenden Körperteil kann dadurch möglichst gering gehalten werden.

Die Markiereinrichtung, insbesondere deren Markierelemente, kann/können am vorstehend erwähnten Halteteil, insbesondere dem Halteelement, festgelegt werden.

Zu diesem Zweck kann die Markiereinrichtung oder die zwei oder mehr Markierelemente ein Befestigungselement aufweisen.

Alternativ kann ein Befestigungselement zum Festlegen am Trägerkörper vorhanden sein.

Als Befestigungselemente können vorliegend beispielsweise Rastelemente, Klemmelemente, Klettbänder oder Magneten zum Einsatz kommen.

Die Erfindung betrifft ferner ein medizinisches Instrumentarium umfassend eine Befestigungseinrichtung. Die eingangs genannte Aufgabe wird erfindungsgemäß durch ein Instrumentarium gelöst, das eine Befestigungseinrichtung der vorstehend genannten Art umfasst und eine optische Erfassungseinheit, mit der Bilder der am Körperteil des Patienten angebrachten Befestigungseinrichtung bei deren Bewegung im Raum erfassbar sind, sowie eine Datenverarbeitungseinrichtung, der von der optischen Erfassungseinheit diesbezügliche Bildsignale bereitstellbar sind, wobei die Datenverarbeitungseinrichtung so ausgebildet und programmiert ist, dass sie anhand der Bildsignale den Trägerkörper in unterschiedliche Verformungsbereiche einteilt, wobei zumindest zwei sich hinsichtlich ihrer Verformung infolge der Bewegung der Befestigungseinrichtung unterscheidende Verformungsbereiche ermittelbar sind. Das Instrumentarium kann den Operateur insbesondere dabei unterstützen, einen oder mehrere Bereiche geringer Verformung zu identifizieren, um daran die Markiereinrichtung oder ein Markierelement festzulegen.

Ein derartiger Bereich verhältnismäßig geringer Verformung kann sich von dem ersten und/oder dem mindestens einen zweiten Dehnungsbereich unterscheiden.

Unter "Verformung" zur Ermittlung der Verformungsbereiche kann jedoch insbesondere eine Dehnung des Trägerkörpers angesehen werden.

Zur Ermittlung der Verformungsbereiche kann die Datenverarbeitungseinrichtung ein Bildverarbeitungsprogramm umfassen, das selbsttätig anhand der Bildsignale der Erfassungseinheit feststellt, welche Bereiche des Trägerkörpers sich in verhältnismäßig geringem Umfang verformen, wenn das Körperteil bewegt wird. Zu diesem Zweck kann das Bildverarbeitungsprogramm beispielsweise ein Finite-Elemente-Verfahren anwenden.

Günstigerweise umfasst das Instrumentarium eine Anzeigeeinheit, an der die Datenverarbeitungseinrichtung ein Bild des Trägerkörpers darstellt, in dem die Verformungsbereiche zur visuellen Unterscheidung voneinander gekennzeichnet sind. Dadurch können die unterschiedlichen Verformungsbereiche vom Operateur einfach erkannt werden.

An der Anzeigeeinheit ist vorzugsweise ein Hinweis ausgebbar, eine Markiereinrichtung oder ein Markierelement an einem Verformungsbereich festzulegen, dessen Verformung geringer ist als die Verformung zumindest eines weiteren Bereiches des Trägerkörpers, wodurch dem Operateur die Positionierung der Markiereinrichtung oder des Markierelementes erleichtert wird.

Die Datenverarbeitungseinrichtung kann beispielsweise eine oder mehrere Farben oder eine Oberflächenstruktur des Trägerkörpers zur Einteilung der Verformungsbereiche heranziehen, insbesondere Farben von Fäden oder eine Oberflächenstruktur infolge einer Bindung von Fäden eines als Textilverbund ausgestalteten oder einen solchen umfassenden Trägerkörpers. Dementsprechend kann die Datenverarbeitungseinrichtung beispielsweise Verformungsbereiche einteilen, in denen sich die Oberfläche eines Gestricks nicht oder nur in geringem Umfang ändert und einen derartigen Bereich als Verformungsbereich geringer Verformbarkeit ansehen. Zu diesem Zweck ist es günstig, wenn der Textilverbund, insbesondere das Gestrick, ein Muster mit hohem Kontrast aufweist. Dieser hohe Kontrast wird zum Beispiel durch die Farbe der zum Einsatz kommenden Fäden, beispielsweise schwarz-weiße Fäden ermöglicht.

Vorzugsweise ist die Datenverarbeitungseinrichtung tragbar und/oder die Anzeigeeinheit und/oder die Erfassungseinheit ist in die Datenverarbeitungseinrichtung integriert. Beispielsweise kommt ein mobiles tragbares Gerät in Gestalt eines Tablet-Computers oder eines Smartphones zum Einsatz.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine perspektivische Darstellung eines medizinischen Navigationssystems, umfassend eine an einem Patienten festgelegte erfindungsgemäße Referenzierungsvorrichtung mit einer erfindungsgemäßen Befestigungseinrichtung;
- Figur 2:: eine Draufsicht auf eine erste Seite der Befestigungseinrichtung aus Figur 1, ausgestaltet als Bandage;
- Figur 3:: eine Draufsicht auf eine zweite Seite der Befestigungseinrichtung, die der ersten Seite gegenüberliegt;
- Figur 4:: schematisch einen Oberschenkel des Patienten, an dem die Referenzierungsvorrichtung festgelegt ist;
- Figur 5:: eine Schnittansicht (teilweise) längs der Linie 5-5 in Figur 4;
- Figur 6:: eine Darstellung entsprechend Figur 5 bei einer andersartigen Ausführungsform einer Befestigungseinrichtung und einer Referenzierungsvorrichtung;
- Figur 7:: eine Draufsicht auf eine weitere Ausführungsform einer Befestigungseinrichtung und einer Referenzierungsvorrichtung und
- Figur 8:: eine Draufsicht auf eine weitere Ausführungsform einer Befestigungseinrichtung.

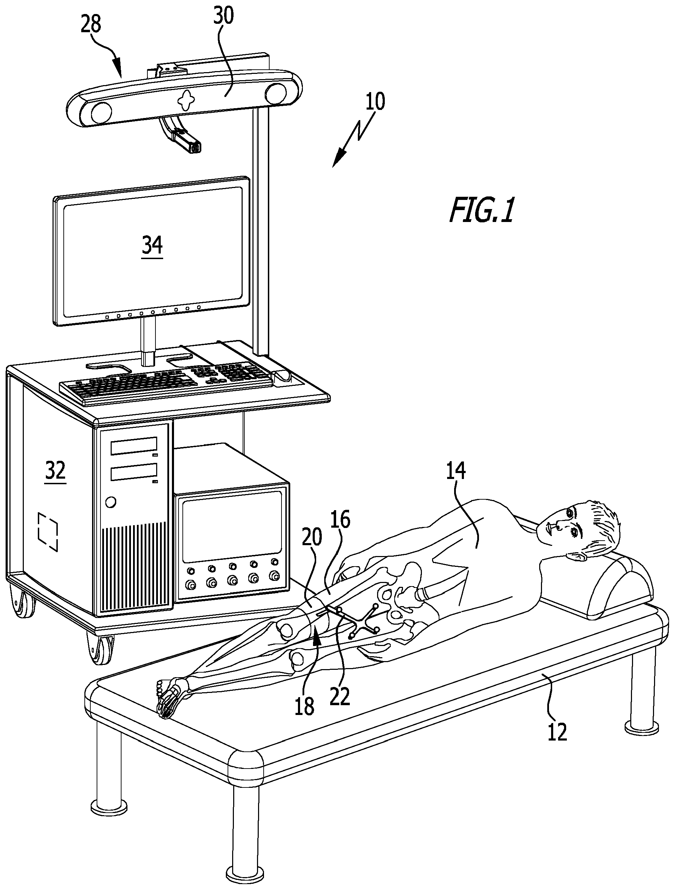

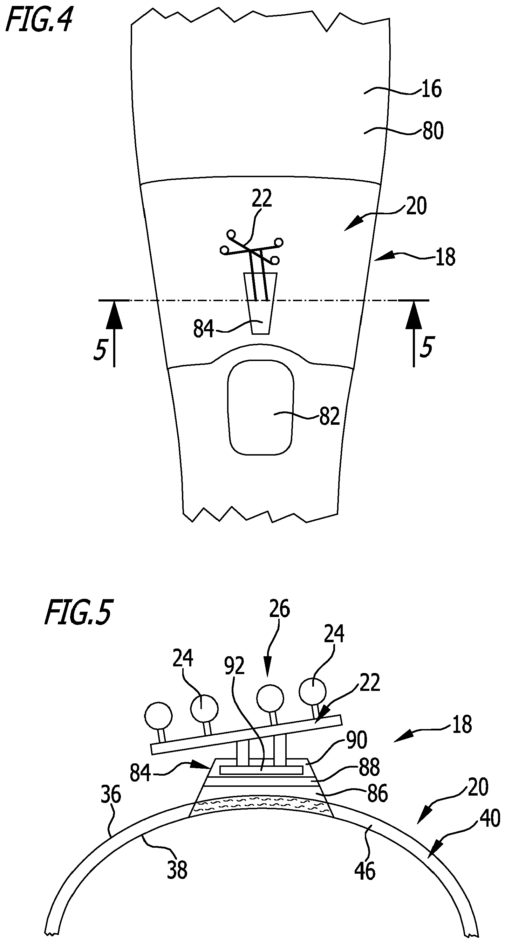

Figur 1 zeigt in perspektivischer Darstellung ein medizinisches Navigationssystem 10 und einen auf einer Liege 12 liegenden Patienten 14, der einen Femur (vorliegend Oberschenkel) 16 aufweist. Das Navigationssystem 10 umfasst eine mit dem Bezugszeichen 18 belegte vorteilhafte Ausführungsform einer erfindungsgemäßen Referenzierungsvorrichtung. Die Referenzierungsvorrichtung 18 weist eine am Femur 16 nichtinvasiv festlegbare vorteilhafte Ausführungsform einer erfindungsgemäßen Befestigungseinrichtung 20 und eine medizinische Markiereinrichtung 22 auf. Die Markiereinrichtung 22 ist ausgestaltet als sogenannter "Rigid Body" mit Markierelementen 24 (Figur 5). Die Markierelemente 24 sind ortsfest relativ zueinander angeordnet und bilden eine starre Markierelementanordnung 26 am Femur 16.

Bewegt sich der Femur 16 im Raum, bewegt sich auch die Markiereinrichtung 22. Von den Markierelementen 24 reflektierte Strahlung, die von einer Nachweiseinrichtung 28 des Navigationssystems 10 emittiert wird, kann von der Nachweiseinrichtung 28 erfasst werden. Zu diesem Zweck umfasst die Nachweiseinrichtung 28 eine Stereokamera 30. Positionsdaten der Nachweiseinrichtung 28 werden einer Datenverarbeitungseinrichtung 32 des Navigationssystems übermittelt. Die Datenverarbeitungseinrichtung 32 berechnet anhand der Positionsdaten die Lage und die Orientierung der Markiereinrichtung 22 und damit des Femurs 16 im Raum. Eine diesbezügliche Information kann an einer Anzeigeeinrichtung 34 des Navigationssystems 10 dargestellt werden.

Das Navigationssystem 10 mit der Referenzierungsvorrichtung 18 kann beispielsweise zur navigationsunterstützten Implantation eines künstlichen Kniegelenkes zum Einsatz kommen. Aus nachfolgend noch ersichtlichen Gründen ist insbesondere ein präoperativer, ein intraoperativer und ein postoperativer Einsatz der Referenzierungsvorrichtung 18 möglich.

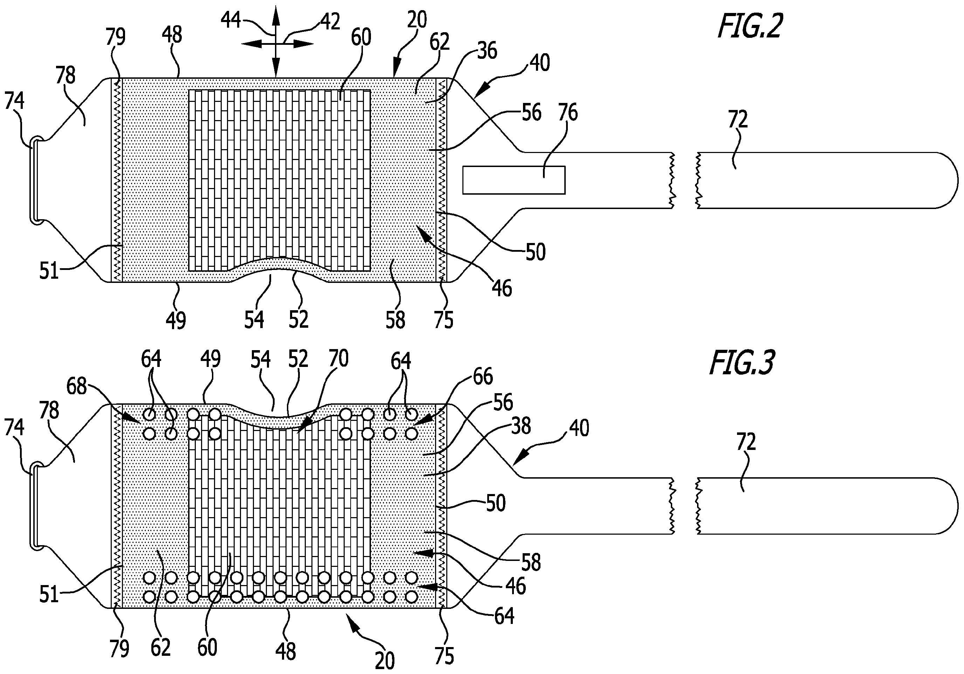

Die Figuren 2 und 3 stellen schematisch die Befestigungseinrichtung 20 in Draufsicht auf eine erste Seite (nachfolgend Oberseite 36) und eine zweite Seite (nachfolgend Unterseite 38) dar. Im bestimmungsgemäßen Gebrauch der Befestigungseinrichtung 20 und der Referenzierungsvorrichtung 18 ist die Unterseite 38 dem Femur 16 zugewandt und die Oberseite 36 dem Femur 16 abgewandt.

Die Befestigungseinrichtung 20 ist vorliegend ausgestaltet als Bandage 40, insbesondere als Kompressionsbandage. Die Bandage 40 umfasst einen in zwei Erstreckungsrichtungen 42, 44, die quer zueinander ausgerichtet sind, flächig erstreckten Trägerkörper 46 für die Markiereinrichtung 22. Der Trägerkörper 46 ist formveränderlich und beim Anlegen der Bandage 40 an den Femur 16 an dessen Kontur anpassbar. Dadurch kann der Trägerkörper 46, wie die Bandage 40 insgesamt, formschlüssig an den Femur 16 angelegt werden und diesen umgeben. Bei Anlage der Bandage 40 am Femur 16 unter Verformung des Trägerkörpers 46 im bestimmungsgemäßen Gebrauch verläuft die Erstreckungsrichtung 42 in Umfangsrichtung des Femurs 16 und die Erstreckungsrichtung 44 in Längsrichtung des Femurs 16.

Der Trägerkörper 46 weist eine in Draufsicht im Wesentlichen rechteckförmige Gestalt auf mit zwei parallel zueinander verlaufenden Längsseiten 48, 49 in Erstreckungsrichtung 42 und Querseiten 50, 51 in Erstreckungsrichtung 44. Die Längsseite 49 umfasst in ihrem mittleren Abschnitt eine bogenförmige Krümmung 52, so dass am Rand des Trägerkörpers 46 im Bereich der Krümmung 52 eine Ausnehmung 54 angeordnet ist.

Der Trägerkörper 46 ist vorliegend ein Textilverbund 56, ausgestaltet als Gestrick 58. Dadurch lässt sich der Trägerkörper 46 und damit die Bandage 40 herstellungstechnisch einfach und kostengünstig fertigen. Das Gestrick 58 weist vorliegend zwei Bereiche unterschiedlicher Bindung auf, wodurch der Trägerkörper 46 aufgrund der unterschiedlichen Bindung und der zum Einsatz kommenden Fäden bereichsweise unterschiedliche Eigenschaften umfasst.

Insbesondere ist das Gestrick 58 so beschaffen, dass der Trägerkörper 46 in der Fläche seiner Erstreckung in zwei quer zueinander ausgerichteten Richtungen elastisch dehnbar ist, insbesondere in der Erstreckungsrichtung 42 und der Erstreckungsrichtung 44. Beim Anlegen der Bandage 40 kann der Trägerkörper 46 dabei gedehnt werden, um den Femur 16 zu komprimieren, worauf nachfolgend noch eingegangen wird.

Das Gestrick 58 weist einen ersten Dehnungsbereich 60 auf, der in Draufsicht auf den Trägerkörper 46 eine im Wesentlichen rechteckförmige Form aufweist. Der Dehnungsbereich 60 befindet sich in der Mitte des Trägerkörpers 46 und ist umgeben von einem zweiten Dehnungsbereich 62. Der Dehnungsbereich 62 fasst den Dehnungsbereich 60 ein und bildet einen Rand für diesen. Ferner bildet der Dehnungsbereich 62 die Längsseiten 48, 49 und die Querseiten 50, 51.

Infolge der Ausgestaltung des Gestricks 58 sind die Dehnungsbereiche 60, 62 durch Stricken herstellungstechnisch einfach und in einem Arbeitsgang miteinander verbunden.

Im vorliegenden Fall ist der erste Dehnungsbereich 60 ein Gestrick mit hoher Zweizugelastizität, beispielsweise mit Köperbindung, das insbesondere elastische Strickfäden aufweist. Der Dehnungsbereich 62 ist ein Gestrick mit Halbschlauchbindung mit Fäden geringerer Elastizität. Im Ergebnis weist dadurch der Dehnungsbereich 60 in beiden Erstreckungsrichtungen 42, 44 (sowie in einer Richtung, die sich aus einer Überlagerung der Erstreckungsrichtungen 42, 44 ergibt und jeweils im Winkel zu diesen in der Fläche des Trägerkörpers 46 ausgerichtet ist) eine größere Dehnbarkeit auf als der Dehnungsbereich 62.

Die Dehnbarkeit des Dehnungsbereiches 60 ist in der Erstreckungsrichtung 44 größer als in der Erstreckungsrichtung 42.

An der Unterseite 38 umfasst der Trägerkörper 46 eine Mehrzahl von Reibwertsteigerungselementen 64 (nachfolgend vereinfachend Reibelemente 64). Die Reibelemente 64 sind vorliegend punktförmig und beispielsweise ungefähr stecknadelkopfgroß. Vorzugsweise sind die Reibelemente 64 aus einem Silikonmaterial gefertigt.

An dem die Längsseite 48 aufweisenden Rand umfasst der Trägerkörper 46 Reibelemente 64. Die Reibelemente 64 verlaufen doppelreihig parallel zur Längsseite 48 und sind gleichmäßig voneinander beabstandet. Die Reibelemente 64 erstrecken sich weitgehend über die gesamte Länge des Trägerkörpers 46 und sind an beiden Dehnungsbereichen 60, 62 angeordnet.

Am gegenüberliegenden, die Längsseite 49 bildenden Rand umfasst der Trägerkörper 46 einen ersten Abschnitt 66 und einen zweiten Abschnitt 68 jeweils mit Reibelementen 64 und einen dazwischen liegenden Abschnitt 70, der frei ist von Reibelementen 64. Der Abschnitt 66 ist in Erstreckungsrichtung 42 zwischen der Ausnehmung 54 und der Querseite 50 angeordnet und der Abschnitt 68 zwischen der Ausnehmung 54 und der Querseite 51. Der Abschnitt 70 erstreckt sich etwas über die Länge der Ausnehmung 54 in der Mitte des Trägerkörpers 46. An jedem Abschnitt 66, 68 verlaufen die Reibelemente 64 doppelreihig parallel zur Längsseite 49 und sind dabei jeweils äquidistant zueinander am Trägerkörper 46 angeordnet.

Die Reibelemente 64 ermöglichen es, die Reibung zwischen der Bandage 40 und dem Femur 16 lokal zu steigern. Im Bereich des Abschnittes 70 wird die Reibung gezielt gering gehalten.

Die Bandage 40 ist eine größenanpassbare Bandage, die von einem in den Figuren 2 und 3 dargestellten geöffneten Lösezustand in einen Anlegezustand überführbar ist, in der die Bandage 40 in sich geschlossen ist (Figuren 1, 4, 5). Zu diesem Zweck weist die Bandage 40 Fixierelemente auf, nämlich einen Gurt 72 und eine mit diesem zusammenwirkende Öse 74. Der Gurt 72 ist an der Querseite 50 über ein Versteifungselement 75 festgelegt und erstreckt sich längs der Erstreckungsrichtung 42 (bei flachgelegter Bandage 40).

Das Versteifungselement 75 umfasst beispielsweise einen Gewebetunnel mit darin angeordneter Schraubenfeder oder Leiste, zum Beispiel aus Kunststoff. Nahe der Querseite 50 ist am Gurt 72 ein Fixierelement vorgesehen in Gestalt eines Klettbandes 76, an dem der Gurt 72 in sich geschlossen haftend befestigt werden kann.

Die Öse 74 ist an einer Seite eines Halteabschnittes 78 befestigt, der an der gegenüberliegenden Seite an der Querseite 51 über ein Versteifungselement 79 mit dem Trägerkörper 46 verbunden ist. Das Versteifungselement 79 entspricht in Form und Funktion dem Versteifungselement 75.

Zum Überführen der Bandage 40 in den Anlegezustand am Femur 16 wird die Unterseite 38 nichtinvasiv auf dessen Haut 80 positioniert (Figur 4). Bevorzugt wird die Bandage 40 in Erstreckungsrichtung 42 gedehnt, entgegen der Elastizität der Dehnungsbereiche 60, 62. Die Bandage 40 wird bevorzugt so am Femur 16 positioniert, dass Weichteilgewebe oberhalb der Kniescheibe 82 in die Ausnehmung 54 eingreift.

Im Anlegezustand wird die Bandage 40 dadurch fixiert, dass der Gurt 72 unter Straffung der Bandage 40 durch die Öse 74 geführt wird und in sich am Klettband 46 befestigt wird. Die Versteifungselemente 75, 79 stellen sicher, dass die Bandage in der Erstreckungsrichtung 44 ihre Form behält und sich der Trägerkörper durch das Straffen nicht verjüngt.

Greift das Weichteilgewebe oberhalb der Kniescheibe 82 in die Ausnehmung 54 ein, ist der Dehnungsbereich 60 insbesondere im Bereich des Abschnittes 70 frei von Reibelementen 64 oberhalb der Sehne des Musculus quadriceps des Femurs 16 angeordnet.

Die Bandage 40 und damit die Referenzierungsvorrichtung 18 ist patienten- und handhabungsfreundlich nichtinvasiv am Femur 16 festlegbar. Im Anlegezustand komprimiert die Bandage 40 den Femur 16. Bewegungen von Haut, Weichteilgewebe und Muskeln relativ zueinander und relativ zum Femurknochen werden dadurch eingeschränkt. Infolgedessen verringert sich auch die Fähigkeit der Bandage 40, sich relativ zum Femur 16 und insbesondere zum Femurknochen zu bewegen.

Auftretende Relativbewegungen von Haut, Weichteilgewebe und Muskeln können außerdem weitgehend innerhalb des Trägerkörpers 46 absorbiert werden. Zu diesem Zweck erweist sich die Fertigung des Trägerkörpers 46 als Gestrick 58 mit den Dehnungsbereichen 60, 62, die sich hinsichtlich ihrer Dehnbarkeit in zumindest einer der Erstreckungsrichtungen 42, 44 unterscheiden, als besonders vorteilhaft. Die Dehnungsbereiche 60, 62 sind so gestrickt, dass Bewegungen innerhalb des Femurs 16 vom Trägerkörper 46 zu einem großen Anteil absorbiert werden. Dabei erfolgt insbesondere am ersten Dehnungsbereich 60 in dessen Mitte (bezogen auf die Erstreckungsrichtung 42), die den Abschnitt 70 frei von Reibelementen 64 aufweist, eine besonders geringe Bewegung innerhalb des Trägerkörpers 46. Die Reibelemente 64 und deren Anordnung am Trägerkörper 46 verbessern über die gezielte Kompression des Femurs 16 die Eigenschaft der Bandage 40, Bewegungen gezielt in den Trägerkörper 46 einzuleiten.

Das Vorsehen des Abschnittes 70 frei von Reibelementen 64 hat wie erwähnt den Vorteil, dass die Reibung zwischen dem Femur 16 und der Bandage 40 lokal geringgehalten werden kann. Beispielsweise reibt die Haut 80 am Abschnitt 70 bei einer Extension und einer Flexion des Beines nur in geringem Ausmaß am Trägerkörper 46, so dass dieser von einer Weichteilverschiebung nicht oder nur in geringem Umfang erfasst wird. Eine Markiereinrichtung an der Oberseite 36 an oder nahe dem Abschnitt 70 kann dadurch eine valide Referenz bilden, deren Position relativ zum Femurknochen auch bei einer Extension oder Flexion des Beines beibehalten werden kann. Zu diesem Zweck ist es auch von Vorteil, dass der Trägerkörper 46 längs der Erstreckungsrichtung 44 eine größere Dehnbarkeit aufweist als längs der Erstreckungsrichtung 42.

Ist die Kniescheibe 82 oder Weichteilgewebe oberhalb derselben im Bereich der Ausnehmung 54 angeordnet und der Abschnitt 70 oberhalb der Sehne des Musculus quadriceps, ist die Bewegung des Trägerkörpers 46 in diesem Bereich besonders gering. Dies ergibt sich auch als Folge der verhältnismäßig geringen Relativbeweglichkeit der Sehne des Musculus quadriceps zu anderem Weichteilgewebe und anderen Muskeln im Femur 16.

Aus diesem Grund weist die Befestigungseinrichtung 20 am Abschnitt 70 ein Halteteil 84 auf, an dem die Markiereinrichtung 22 festlegbar ist. Das Halteteil 84 ist in den Figuren 1 bis 3 nicht dargestellt.

Das Halteteil 84 weist eine prismatische, ungefähr pyramidenstumpfförmige Gestalt auf und ist am Trägerkörper 46, insbesondere am Dehnungsbereich 60 festgelegt. Das Halteteil 84 weist einen Mehrschichtaufbau auf mit einer ersten Schicht 86, einer zweiten Schicht 88 und einer dritten Schicht 90. Die Schichten 86 bis 90 sind vorliegend beispielsweise aus einem Silikonmaterial gefertigt. Günstig ist es, wenn sie unterschiedliche Verformbarkeiten aufweisen, wobei bevorzugt die Verformbarkeit von der Schicht 86 über die Schicht 88 bis zur Schicht 90 abnimmt. Am Trägerkörper 46 noch auftretende Bewegungen können dadurch innerhalb des Halteteils 84 absorbiert werden und werden nur in geringem Umfang oder nicht auf die Markiereinrichtung 22 übertragen. Über die Schicht 86 greift das Halteteil 84 in das Gestrick 58 des Trägerkörpers 46 ein und ist teilweise in dieses eingebettet, wodurch das Halteteil 84 am Trägerkörper 46 festgelegt ist.

An der Schicht 90 ist ein Halteelement 92 festgelegt, an dem die Markiereinrichtung 22 festgelegt oder insbesondere lösbar festlegbar ist. Das Halteelement 92 ist vorliegend in die Schicht 90 eingebettet, beispielsweise in diese eingegossen. Die Markierelementanordnung 26 kann zum Beispiel durch Verschrauben, Verklemmen, Verrasten, Verkleben, über eine Klettverbindung oder dergleichen kraft- und/oder formschlüssig am Halteelement 92 festgelegt werden.

Durch die Möglichkeit der nichtinvasiven Befestigung der Referenzierungsvorrichtung 18 am Patienten 14 ergibt sich eine patientenfreundliche Möglichkeit, ein Körperteil, wie beispielsweise den Femur 16, mit dem Navigationssystem 10 im Raum zu verfolgen. Wie erwähnt ist ein präoperativer, eine intraoperativer und ein postoperativer Einsatz möglich. Trotz der patientenschonenden Befestigung kann eine hinreichend genaue Referenz durch die Ausgestaltung der Befestigungseinrichtung 20 wie vorstehend erläutert erzielt werden.

Nachfolgend wird auf weitere bevorzugte Ausführungsformen erfindungsgemäßer Befestigungseinrichtungen und Referenzierungsvorrichtungen eingegangen. Für gleiche oder gleichwirkende Merkmale und Bauteile werden dabei weitgehend identische Bezugszeichen verwendet.

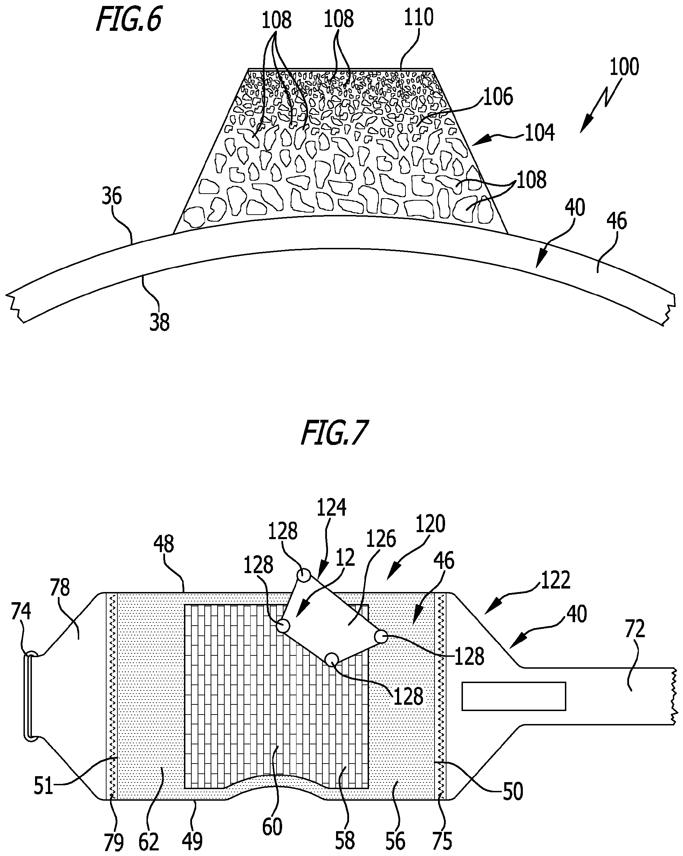

Figur 6 zeigt in einer der Figur 5 ähnlichen Weise schematisch eine Schnittdarstellung durch eine zweite bevorzugte Ausführungsform einer Befestigungseinrichtung mit Bezugszeichen 100. Die Befestigungseinrichtung 100 umfasst die Bandage 40. An der Bandage 40 ist ein Halteteil 104 befestigt, das in seiner Funktion dem Halteelement 92 entspricht. Am Halteteil 104 kann eine in der Zeichnung nicht gezeigte Markiereinrichtung festgelegt oder insbesondere lösbar befestigt werden, zum Beispiel durch Verschrauben, Verklemmen, Verrasten, Verkleben oder über eine Klettverbindung.

Das Halteteil 104 ist an der Oberseite 36 festgelegt und umfasst einen porösen Körper 106. Der Körper 106 weist eine Vielzahl von Hohlräumen 108 auf. Dabei ist der Körper 106 so ausgestaltet, dass die mittlere Größe der Hohlräume 108 von der dem Trägerkörper 46 zugewandten Seite des Halteteils 104 bis zu der dem Trägerkörper 46 abgewandten Seite des Halteteils 104 kleiner wird. Insbesondere kann die mittlere Größe kontinuierlich kleiner werden. Dadurch fällt die Verformbarkeit des Halteteils 104 von der dem Trägerkörper 46 zugewandten Seite zu der dem Trägerkörper 46 abgewandten Seite ab, und das Halteteil 104 ist am Trägerkörper 46 weicher als im Abstand zu diesem. Wie beim Halteteil 84 ist dadurch die Möglichkeit gegeben, Bewegungen des Trägerkörpers 46 innerhalb des Halteteils 104 zu absorbieren, wodurch diese nicht oder nur in geringem Umfang an eine Markiereinrichtung übertragen werden, die an einer Ankoppelstelle 110 an der dem Trägerkörper 46 abgewandten Seite des Halteteils 104 festlegbar ist (nicht gezeigt). Beispielsweise kommt die Markiereinrichtung 22 zum Einsatz.

Figur 7 zeigt eine weitere Referenzierungsvorrichtung 120 mit einer Befestigungseinrichtung 122 und einer Markiereinrichtung 124. Die Befestigungseinrichtung 122 ist, abgesehen vom nicht vorhandenen Halteteil 84, identisch zur Befestigungseinrichtung 20 ausgestaltet.

Die Markiereinrichtung 124 ist vorliegend von kompakter Bauform und weist ein plattenförmiges Tragelement 126 von ungefähr trapezförmiger Gestalt auf und an dessen Ecken festgelegte Markierelemente 128. Diese bilden eine starre Markierelementanordnung 126. Die Markiereinrichtung 124 hat insbesondere eine geringe Bauhöhe und ist verhältnismäßig leicht. Eine Relativbewegung der Markiereinrichtung 124 und der Befestigungseinrichtung 122 ist aufgrund verringerter Trägheit besonders gering. Aufgrund der geringen Bauhöhe führen selbst Relativbewegungen der Markiereinrichtung 124 und des Trägerkörpers 46 nur zu geringen Verfälschungen von Positionsdaten, wodurch mit der Referenzierungsvorrichtung 120 eine hohe Genauigkeit von Positionsdaten bei der Verfolgung des Femurs 16 im Raum erzielbar ist.

Die Markiereinrichtung 124 kann am Trägerkörper 46 konstruktiv einfach festgelegt werden, beispielsweise durch Verklebung oder über eine Klettverbindung.

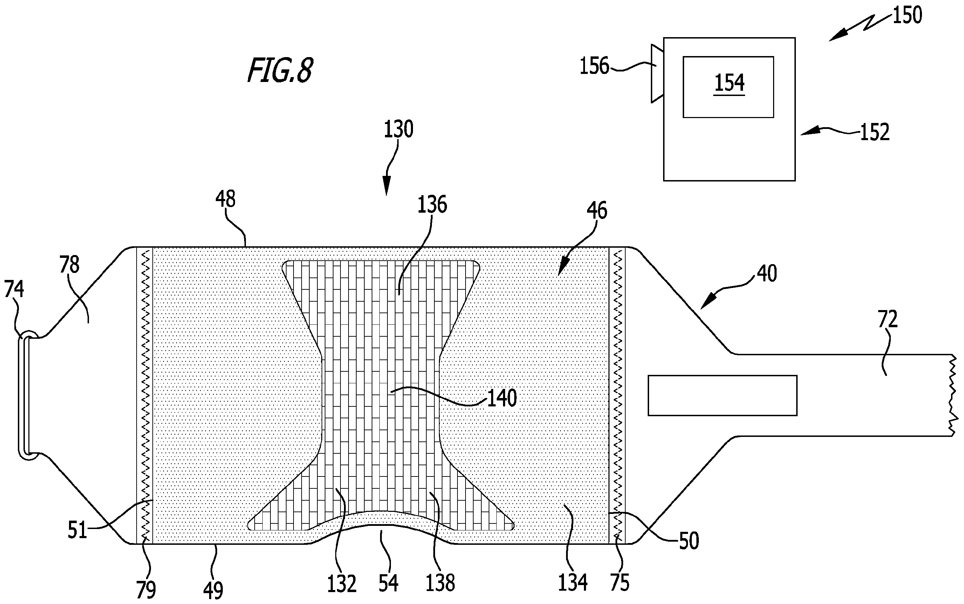

Figur 8 stellt in einer der Figur 2 entsprechenden Weise eine weitere Befestigungseinrichtung 130 dar. Die Befestigungseinrichtung 130 ist weitgehend identisch zur Befestigungseinrichtung 20. Sie kann das Halteteil 84 oder das Halteteil 104 aufweisen oder wie die Befestigungseinrichtung 122 kein Halteteil umfassen. Die Markiereinrichtung 124 kann beispielsweise mit der Befestigungseinrichtung 130 zur Bildung einer Referenzierungsvorrichtung zusammenwirken.

Bei der Befestigungseinrichtung 130 sind zwei Dehnungsbereiche 132, 134 vorhanden, die den Dehnungsbereichen 60, 62 entsprechen. Der Dehnungsbereich 134 umgibt den Dehnungsbereich 132. Der Dehnungsbereich 132 weist in Draufsicht auf den Trägerkörper 46 eine taillierte Kontur auf mit zwei im Abstand zueinander angeordneten Verbreiterungsabschnitten 136, 138. Zwischen diesen ist ein Verschmälerungsabschnitt 140 angeordnet. Die Abschnitte 136 bis 140 sind längs der Erstreckungsrichtung 42 in der Mitte zwischen den Querseiten 50, 51 angeordnet. In der Erstreckungsrichtung 44 ist der Verbreitungsabschnitt 136 der Längsseite 48 benachbart und der Verbreiterungsabschnitt 138 der Längsseite 49.

Die Befestigungseinrichtung 130 und auch die Befestigungseinrichtung 122 weisen an ihrer jeweiligen, in der Zeichnung nicht gezeigten Unterseite ebenfalls Reibelemente auf.

Die bereits im Zusammenhang mit der Erläuterung der Befestigungseinrichtung 20 erzielbaren Vorteile können unter Einsatz der Befestigungseinrichtungen 100, 122 und 130 ebenfalls erzielt werden.

Über die Befestigungseinrichtung 130 hinaus zeigt Figur 8 ein medizinisches Instrumentarium 150, welches vorliegend die Befestigungseinrichtung 130 umfasst sowie eine Datenverarbeitungseinrichtung 152. Die Datenverarbeitungseinrichtung 152 ist bevorzugt handhaltbar und tragbar, beispielsweise in Gestalt eines Tablet-Computers. Dementsprechend kann die Datenverarbeitungseinrichtung 152 vorzugsweise eine Anzeigeeinheit 154 als integralen Bestandteil umfassen.

Ferner ist in die Datenverarbeitungseinrichtung 152 eine in Figur 8 schematisch dargestellte optische Erfassungseinheit 156 integriert, die insbesondere eine Digitalkamera umfasst.

Die Erfassungseinheit 156 wird in Anwendung des Instrumentariums 150 auf die am Femur 16 festgelegte Bandage 40, insbesondere deren Trägerkörper 46, gerichtet. Bildsignale, die von der Erfassungseinheit bereitgestellt werden, werden von der Datenverarbeitungseinrichtung 152 dahingehend untersucht, ob der Trägerkörper 46 in unterschiedliche Verformungsbereiche eingeteilt werden kann. Infolge einer Bewegung des Femurs 16 und damit verbundene Verschiebungen von Haut, Weichteilgewebe, Muskeln und Femurknochen relativ zueinander sowie einer möglichen Bewegung der Bandage 40 am Femur 16 kann der Trägerkörper 46 Bereiche unterschiedlicher Verformung aufweisen. Diese Bereiche können sich von den Dehnungsbereichen 132, 134 unterscheiden. Bei der Befestigungseinrichtung 130 ermittelt die Datenverarbeitungseinrichtung 152 beispielsweise, dass sich jedoch am Dehnungsbereich 132 nahe der Ausnehmung 54 ein Bereich des Trägerkörpers 46 mit geringer Verformung befindet, wie dies bereits weiter oben erläutert wurde.

Zur Ermittlung der Verformungsbereiche kann die Datenverarbeitungseinrichtung 152 beispielsweise eine oder mehrere Farben oder eine Oberflächenstruktur des Trägerkörpers 46 heranziehen, beispielsweise Farben von Fäden des Trägerkörpers 46 oder eine Oberflächenstruktur infolge der Bindung, beispielsweise der sich unterscheidenden Bindungen der Verformungsbereiche 136, 140. Dementsprechend kann die Datenverarbeitungseinrichtung 152 ein Bildbearbeitungsprogramm umfassen, welches selbsttätig anhand der Bildsignale ermittelt, welcher Bereich des Trägerkörpers 46 sich in verhältnismäßig geringem Umfang verformt. Beispielsweise wird zu diesem Zweck ein Finite-Elemente-Verfahren angewandt.

An der Anzeigeeinheit 154 kann der Bedienperson ein Bild des Trägerkörpers 46 angezeigt werden. In diesem Bild sind die unterschiedlichen Verformungsbereiche vorzugsweise gekennzeichnet. Günstig ist es, wenn der Bedienperson ein Hinweis bereitgestellt wird, wo am Trägerkörper 46 eine Markiereinrichtung, zum Beispiel die Markiereinrichtung 124, vorteilhafterweise platziert werden soll, um eine möglichst stabile Referenz zu erhalten.

Die integrierte Datenverarbeitungseinrichtung 152 kann selbstverständlich auch mit den Befestigungseinrichtungen 100, 122 und 20 zum Einsatz kommen. Beispielsweise wurde bei der Referenzierungsvorrichtung 120 die Markiereinrichtung 124 an der in Figur 7 dargestellten Position an der Befestigungseinrichtung 122 platziert, weil sich diese Position als geeignet für eine stabile Referenz erwiesen hat.

## Patentansprüche

1. Medizinische Befestigungseinrichtung zum nichtinvasiven Befestigen einer zwei oder mehr Markierelemente (24; 128) aufweisenden medizinischen Markiereinrichtung (22; 124) an einem Körperteil (16) eines Patienten (14), insbesondere an einem Oberschenkel, wobei die Befestigungseinrichtung (20; 100; 122; 130) einen in zwei im Winkel zueinander ausgerichteten Erstreckungsrichtungen (42, 44) flächig erstreckten, in seiner Form an die Kontur des Körperteils (16) anpassbaren Trägerkörper (46) umfasst, an dem die Markiereinrichtung (22; 124) festgelegt oder festlegbar ist, wobei der Trägerkörper (46) in der Fläche seiner Erstreckung elastisch dehnbar ist und einen ersten Dehnungsbereich (60; 132) und zumindest einen zweiten Dehnungsbereich (62; 134) umfasst, dessen Dehnbarkeit in zumindest einer Richtung (42, 44) in der Fläche der Erstreckung des Trägerkörpers (46) geringer ist als diejenige des ersten Dehnungsbereiches (60; 132), wobei die Befestigungseinrichtung (20; 100) mindestens ein Halteteil (84; 104) umfasst, das am Trägerkörper (46) festgelegt ist, und wobei die Markiereinrichtung (22) oder ein Markierelement der Markiereinrichtung (22) an einer dem Trägerkörper (46) abgewandten Seite des Halteteils (84; 104) kraft- und/oder formschlüssig festgelegt oder festlegbar ist.

2. Befestigungseinrichtung nach Anspruch 1, **dadurch gekennzeichnet, dass** die Dehnbarkeit des mindestens einen zweiten Dehnungsbereiches (62; 134) in zwei im Winkel und insbesondere quer zueinander ausgerichteten Richtungen (42, 44) in der Fläche der Erstreckung des Trägerkörpers (46) geringer ist als diejenige des ersten Dehnungsbereiches (60; 132).

3. Befestigungseinrichtung nach einem der voranstehenden Ansprüche, **dadurch gekennzeichnet, dass** der mindestens eine zweite Dehnungsbereich (62; 134) den ersten Dehnungsbereich (60; 132) umgibt und/oder dass der Trägerkörper (46) in Draufsicht eine rechteckige oder im Wesentlichen rechteckige Kontur aufweist.

4. Befestigungseinrichtung nach einem der voranstehenden Ansprüche, **dadurch gekennzeichnet, dass** der Trägerkörper (46) ein Textilverbund (56) in Form eines Gestricks (58), eines Gewirks oder eines Gewebes ist oder dass der Trägerkörper (46) einen solchen Textilverbund (56) umfasst, vorzugsweise dass der erste Dehnungsbereich (60; 132) und der mindestens eine zweite Dehnungsbereich (62; 136) miteinander durch Stricken, Wirken oder Weben verbunden sind.

5. Befestigungseinrichtung nach Anspruch 4, **dadurch gekennzeichnet, dass** die unterschiedliche Dehnbarkeit des ersten Dehnungsbereiches (60; 132) und des mindestens einen zweiten Dehnungsbereiches (62; 134) durch die Art des Gestricks, des Gewirks oder des Gewebes bereitgestellt ist, insbesondere durch die Verknüpfungsart und/oder die Anzahl und/oder Beschaffenheit der Fäden des Textilverbundes (56).

6. Befestigungseinrichtung nach Anspruch 4 oder 5, **dadurch gekennzeichnet, dass** der erste Dehnungsbereich (60; 132) ein Gestrick (58) mit Köperbindung ist oder umfasst und/oder dass der mindestens eine zweite Dehnungsbereich (62; 134) ein Gestrick (58) mit Halbschlauchbindung ist oder umfasst.

7. Befestigungseinrichtung nach einem der voranstehenden Ansprüche, **dadurch gekennzeichnet, dass** die Befestigungseinrichtung (20; 100; 122; 130) als Bandage (40) ausgestaltet ist oder eine solche umfasst, die von einem geöffneten Lösezustand in einen in sich geschlossenen Anlegezustand überführbar ist und die ein oder mehrere Fixierelemente (72, 74, 76) zum Fixieren der Bandage im Anlegezustand aufweist, vorzugsweise dass die Länge der Bandage (40) im Anlegezustand für einen formschlüssigen Sitz am Körperteil (16) anpassbar ist.

8. Befestigungseinrichtung nach einem der Ansprüche 1 bis 6, **dadurch gekennzeichnet, dass** die Befestigungseinrichtung als in sich geschlossene Schlauchbandage ausgestaltet ist oder eine solche umfasst.

9. Befestigungseinrichtung nach einem der voranstehenden Ansprüche, **dadurch gekennzeichnet, dass** der Trägerkörper (46) auf seiner dem Körperteil (16) zugewandten Seite (38) Reibwertsteigerungselemente (64) umfasst.

10. Befestigungseinrichtung nach einem der voranstehenden Ansprüche, **dadurch gekennzeichnet, dass** das mindestens eine Halteteil (84; 104) verformbar ausgestaltet ist, wobei vorzugsweise die Verformbarkeit an einer dem Trägerkörper (46) zugewandten Seite des Halteteils (84; 104) größer ist als an einer dem Trägerkörper (46) abgewandten Seite des Halteteils (84; 104).

11. Befestigungseinrichtung nach Anspruch 10, **dadurch gekennzeichnet, dass** das Halteteil (84) einen Schichtaufbau aufweist mit zwei oder mehr übereinanderliegenden Schichten (86, 88, 90), wobei die Schichten (86, 88, 90) eine unterschiedliche Verformbarkeit aufweisen.

12. Befestigungseinrichtung nach einem der voranstehenden Ansprüche, **dadurch gekennzeichnet, dass** das Halteteil (104) eine poröse, Hohlräume (108) umfassende Struktur aufweist, wobei eine mittlere Größe von Hohlräumen (108) im Halteteil (104) an dessen dem Trägerkörper (46) abgewandten Seite geringer ist als an dessen dem Trägerkörper (46) zugewandten Seite.

13. Medizinische Referenzierungsvorrichtung, umfassend eine Markiereinrichtung (22; 124) mit mindestens zwei oder mehr chirurgischen Markierelementen (24, 128), die zum Reflektieren und/oder Emittieren von Strahlung ausgebildet sind und eine Markierelementanordnung (26) ausbilden zur Definition einer Referenz am Körper eines Patienten (14), sowie umfassend eine Befestigungseinrichtung (20; 122) nach einem der voranstehenden Ansprüche, wobei die Markiereinrichtung (22; 124) an der Befestigungseinrichtung (20; 122), bevorzugt lösbar, festlegbar oder festgelegt ist.

14. Referenzierungsvorrichtung nach Anspruch 13, **dadurch gekennzeichnet, dass** die Markierelementanordnung (26; 129) starr ausgebildet ist, wobei die Markierelemente (22; 128) fest miteinander verbunden sind und gemeinsam an der Befestigungseinrichtung (20; 122) festlegbar sind, oder dass die Markierelementanordnung nicht-starr ausgebildet ist, wobei zwei oder mehr Markierelemente positionsveränderlich zueinander sind.

15. Medizinisches Instrumentarium umfassend eine Befestigungseinrichtung nach einem der Ansprüche 1 bis 12, eine optische Erfassungseinheit (156), mit der Bilder der am Körperteil (16) des Patienten (14) angebrachten Befestigungseinrichtung (20; 100; 122; 130) bei deren Bewegung im Raum erfassbar sind, sowie eine Datenverarbeitungseinrichtung (152), der von der optischen Erfassungseinheit (156) diesbezügliche Bildsignale bereitstellbar sind, wobei die Datenverarbeitungseinrichtung (152) so ausgebildet und programmiert ist, dass sie anhand der Bildsignale den Trägerkörper (46) in unterschiedliche Verformungsbereiche einteilt, wobei zumindest zwei sich hinsichtlich ihrer Verformung infolge der Bewegung der Befestigungseinrichtung (20; 100; 122; 130) unterscheidende Verformungsbereiche ermittelbar sind.

## Claims

1. Medical fastening device for noninvasively fastening a medical marking device (22; 124) comprising two or more marking elements (24; 128) to a body part (16) of a patient (14), in particular, to a thigh, the fastening device (20; 100; 122; 130) comprising a support body (46) which extends over a surface in two directions of extent (42, 44) aligned at an angle to each other and is adaptable in its shape to the contour of the body part (16), to which the marking device (22; 124) is fixed or fixable, wherein the support body (46) is elastically stretchable in the surface of its extent and comprises a first stretch area (60; 132) and at least one second stretch area (62; 134), the stretchability of which in at least one direction (42, 44) in the surface of the extent of the support body (46) is less than that of the first stretch area (60; 132), wherein the fastening device (20; 100) comprises at least one holding part (84; 104), which is fixed to the support body (46), and wherein the marking device (22) or a marking element of the marking device (22) is releasably fixed or fixable with force and/or positive locking on a side of the holding part (84; 104) facing away from the support body (46).

2. Fastening device in accordance with claim 1, **characterized in that** the stretchability of the least one second stretch area (62; 134) in two directions (42, 44) aligned at an angle and, in particular, transversely to each other in the surface of the extent of the support body (46) is less than that of the first stretch area (60; 132).

3. Fastening device in accordance with any one of the preceding claims, **characterized in that** the at least one second stretch area (62; 134) surrounds the first stretch area (60; 132) and/or **in that** the support body (46), in a plan view, has a rectangular or substantially rectangular contour.

4. Fastening device in accordance with any one of the preceding claims, **characterized in that** the support body (46) is a textile material (56) in the form of a knitted fabric (58), a warp-knitted fabric or a woven fabric or **in that** the support body (46) comprises such a textile material (56), preferably **in that** the first stretch area (60; 132) and the at least one second stretch area (62; 136) are joined to each other by knitting, warp-knitting or weaving.

5. Fastening device in accordance with claim 4, **characterized in that** the different stretchability of the first stretch area (60; 132) and the at least one second stretch area (62; 134) is provided by the type of knitted fabric, warp-knitted fabric or woven fabric, in particular, by the type of linking and/or the number and/or structure of the threads of the textile material (56).

6. Fastening device in accordance with claim 4 or 5, **characterized in that** the first stretch area (60; 132) is or comprises a knitted fabric (58) with twill weave and/or **in that** the at least one second stretch area (62; 134) is or comprises a knitted fabric (58) with semi-tubular weave.

7. Fastening device in accordance with any one of the preceding claims, **characterized in that** the fastening device (20; 100; 122; 130) is configured as bandage (40) or comprises such a bandage (40), which is transferable from an open released state to an applied state closed within itself, and which comprises one or more fixing elements (72, 74, 76) for fixing the bandage in the applied state, preferably **in that** the length of the bandage (40) in the applied state is adaptable for a positive fit on the body part (16).

8. Fastening device in accordance with any one of claims 1 to 6, **characterized in that** the fastening device is configured as tubular bandage closed within itself or comprises such a tubular bandage closed within itself.

9. Fastening device in accordance with any one of the preceding claims, **characterized in that** the support body (46) has friction coefficient increasing elements (64) on its side (38) facing the body part (16).

10. Fastening device in accordance with any one of the preceding claims, **characterized in that** the at least one holding part (84; 104) is of deformable configuration, the deformability at a side of the holding part (84; 104) facing the support body (46) preferably being greater than at a side of the holding part (84; 104) facing away from the support body (46).

11. Fastening device in accordance with claim 10, **characterized in that** the holding part (84) has a layer construction with two or more layers (86, 88, 90) lying one on the other, with the layers (86, 88, 90) having a different deformability.

12. Fastening device in accordance with any one of the preceding claims, **characterized in that** the holding part (104) has a porous structure comprising cavities (108), an average size of cavities (108) in the holding part (104) being smaller at its side facing away from the support body (46) than at its side facing the support body (46).

13. Medical referencing device, comprising a marking device (22; 124) with at least two or more surgical marking elements (24, 128), which are configured to reflect and/or emit radiation and form a marking element array (26) for defining a reference on the body of a patient (14), and comprising a fastening device (20; 122) in accordance with any one of the preceding claims, the marking device (22; 124) being fixable or fixed, preferably releasably, to the fastening device (20; 122).

14. Referencing device in accordance with claim 13, **characterized in that** the marking element array (26; 129) is of rigid construction, the marking elements (22; 128) being firmly connected to one another and jointly fixable to the fastening device (20; 122), or **in that** the marking element array is of non-rigid construction, two or more marking elements being changeable in position relative to one another.

15. Medical instrumentation comprising a fastening device in accordance with any one of claims 1 to 12, an optical detection unit (156) with which images of the fastening device (20; 100; 122; 130) attached to the body part (16) of the patient (14) are detectable upon movement of the fastening device in space, and a data processing device (152) adapted to be provided with image signals relating thereto by the optical detection unit (156), the data processing device (152) being so configured and programmed that on the basis of the image signals it divides the support body (46) into different deformation areas, with at least two deformation areas differing with respect to their deformation as a result of the movement of the fastening device (20; 100; 122; 130) being determinable.

## Revendications

1. Dispositif de fixation médical pour fixer de manière non invasive un dispositif médical de repère (22; 124) comportant deux éléments de repère (24; 128) ou davantage, sur une partie de corps (16) d'un patient (14), notamment sur la cuisse,
le dispositif de fixation (20; 100; 122; 130) comprenant un corps de support (46), qui s'étend à plat dans deux directions d'étendue (42, 44) orientées de manière à former un angle entre-elles, qui, dans sa forme, peut être adapté au contour de la partie de corps (16), et sur lequel est fixé ou peut être fixé le dispositif de repère (22; 124),
le corps de support (46) étant extensible de manière élastique dans la surface de son étendue, et présentant une première zone extensible (60; 132) et au moins une deuxième zone extensible (62; 134) dont la capacité d'extensibilité dans au moins une direction (42, 44) dans la surface de l'étendue du corps de support (46), est moindre que celle de la première zone extensible (60; 132),
le dispositif de fixation (20; 100) comprenant au moins une pièce de maintien (84; 104), qui est fixée au corps de support (46), et
le dispositif de repère (22) ou un élément de repère du dispositif de repère (22) étant fixé ou pouvant être fixé par liaison de force et/ou complémentarité de forme, sur un côté de la pièce de maintien (84; 104), qui est opposé à celui dirigé vers le corps de support (46) .

2. Dispositif de fixation selon la revendication 1, **caractérisé en ce que** la capacité d'extensibilité dans la surface de l'étendue du corps de support (46), de ladite au moins une deuxième zone extensible (62; 134) est moindre que celle de la première zone extensible (60; 132) dans deux directions (42, 44) orientées de manière à former un angle entre-elles, et notamment orientées transversalement l'une par rapport à l'autre.

3. Dispositif de fixation selon l'une des revendications précédentes, **caractérisé en ce que** ladite au moins une deuxième zone extensible (62; 134) entoure la première zone extensible (60; 132), et/ou **en ce que** le corps de support (46) présente en vue en plan, un contour rectangulaire ou sensiblement rectangulaire.

4. Dispositif de fixation selon l'une des revendications précédentes, **caractérisé en ce que** le corps de support (46) est un composé textile (56) sous la forme d'un tricot (58), d'un tissu à mailles ou d'un tissu, ou bien **en ce que** le corps de support (46) comprend un tel composé textile (56), de préférence **en ce que** la première zone extensible (60; 132) et ladite au moins une deuxième zone extensible (62; 134) sont reliées mutuellement par tricotage, maillage ou tissage.

5. Dispositif de fixation selon la revendication 4, **caractérisé en ce que** la capacité d'extensibilité différente de la première zone extensible (60; 132) et de ladite au moins une deuxième zone extensible (62; 134) est donnée par le type du tricot, du tissu à mailles ou du tissu, notamment par le type d'entrelacement et/ou le nombre et/ou la nature des fils du composé textile (56).

6. Dispositif de fixation selon la revendication 4 ou la revendication 5, **caractérisé en ce que** la première zone extensible (60; 132) est ou comprend un tricot (58) à armure croisée, et/ou **en ce que** ladite au moins une deuxième zone extensible (62; 134) est un tricot (58) à armure semi-tubulaire.

7. Dispositif de fixation selon l'une des revendications précédentes, **caractérisé en ce que** le dispositif de fixation (20; 100; 122; 130) est configuré sous forme de bandage (40) ou comprend un tel bandage, qui peut être transféré d'un état libre ouvert à un état appliqué fermé, et qui comporte un ou plusieurs éléments de fixation (72, 74, 76) pour fixer le bandage dans l'état appliqué, de préférence **en ce que** la longueur du bandage (40) peut être ajustée dans l'état appliqué pour obtenir une assise par complémentarité de formes sur la partie de corps (16) considérée.

8. Dispositif de fixation selon l'une des revendications 1 à 6, **caractérisé en ce que** le dispositif de fixation est configuré sous forme de bandage-chaussette fermé en soi, ou comprend un tel bandage-chaussette.

9. Dispositif de fixation selon l'une des revendications précédentes, **caractérisé en ce que** le corps de support (46) comporte sur son côté (38) dirigé vers la partie de corps (16), des éléments d'augmentation de la friction (64) .

10. Dispositif de fixation selon l'une des revendications précédentes, **caractérisé en ce que** ladite au moins une pièce de maintien (84 ; 104) est d'une configuration déformable, la déformabilité étant de préférence plus grande sur un côté de la pièce de maintien (84; 104), qui est dirigé vers le corps de support (46), que sur un côté de la pièce de maintien (84; 104), qui est éloigné du corps de support (46).

11. Dispositif de fixation selon la revendication 10, **caractérisé en ce que** la pièce de maintien (84) présente une structure à couches avec deux ou davantage de couches superposées (86, 88, 90), les couches (86, 88, 90) présentant une déformabilité différente.

12. Dispositif de fixation selon l'une des revendications précédentes, **caractérisé en ce que** la pièce de maintien (104) présente une structure poreuse, qui comprend des cavités (108), une grandeur moyenne des cavités (108) dans la pièce de maintien (104), sur son coté éloigné du corps de support (46), étant moindre que sur son côté dirigé vers le corps de support (46).

13. Dispositif médical de détermination d'une référence, comprenant un dispositif de repère (22; 124) comportant au moins deux éléments de repère chirurgicaux (24; 128) ou davantage, qui sont conçus pour la réflexion et/ou l'émission de rayonnements et forment un agencement d'éléments de repère (26) pour la définition d'une référence sur le corps d'un patient (14), et comprenant également un dispositif de fixation (20; 122) selon l'une des revendications précédentes, le dispositif de repère (22; 124) étant fixé ou pouvant être fixé, de préférence de manière amovible, sur le dispositif de fixation (20; 122).

14. Dispositif de détermination d'une référence selon la revendication 13, **caractérisé en ce que** l'agencement d'éléments de repère (26; 129) est d'une configuration rigide, les éléments de repère (22; 128) étant reliés mutuellement de manière fixe et pouvant être fixés en commun sur le dispositif de fixation (20; 122), ou bien **en ce que** l'agencement d'éléments de repère n'est pas de configuration rigide, deux éléments de repère ou davantage étant de position variable les uns par rapport aux autres.

15. Instrumentation médicale comprenant un dispositif de fixation selon l'une des revendications 1 à 12, une unité de relevé optique (156), qui permet de relever des images du dispositif de fixation (20; 100; 122; 130) rapporté sur la partie du corps (16) du patient (14), lors du mouvement de celui-ci dans l'espace, ainsi qu'un dispositif de traitement de données (152) auquel peuvent être fournis des signaux d'image à cet égard par le dispositif de relevé optique (156), le dispositif de traitement de données (152) étant conçu et programmé de manière à subdiviser le corps de support (46) en différentes zones de déformation au regard des signaux d'image, au moins deux zones de déformation se différenciant quant à leur déformation suite au mouvement du dispositif de fixation (20; 100; 122; 130), étant susceptibles d'être déterminées.
